# EUROPEAN PATENT APPLICATION

(11) **EP 1 769 781 A1**
(43) Date of publication of application: **04.04.2007**
(21) Application number: 06254997.7
(22) Date of filing: 27.09.2006
(51) Int. Cl.: A61F 13/15

(54) **Disposable sanitary napkin with pull-tab and method for the manufacture thereof**

(30) Priority: 28.09.2005 US 721362 P
(71) Applicant: JOHNSON & JOHNSON INC., Montreal, Quebec H1N 2G4 (CA)
(72) Inventor: Wetter, Alexandre, Montréal, Québec H2T 2G1 (CA); Joubert, Stéphane, Mirabel, Québec J7N 2T6 (CA); Canuel, Louis, Repentigny, Québec J6A 5V6 (CA)
(74) Representative: Kirsch, Susan Edith

(57) **Abstract**

The invention provides a disposable sanitary napkin. The sanitary napkin comprises: a main body; an adhesive fastener; at least one peelable protective layer; and at least one pull-tab. The main body, which is adapted for absorbing bodily exudates, also includes: an upper surface, which faces toward the wearer when the napkin is in use; a lower surface, which faces away from the user; a longitudinally extending centerline; a transversally extending centerline; and a peripheral edge. The adhesive fastener is covered by the peelable protective layer prior to the napkin's use. The pull tab contacts the peelable protective layer to promote separation of the peelable protective layer from the adhesive fastener when the pull tab is being pulled.

## Description

### Cross Reference to Related Applications

This application claims priority to Application No. 60/721,362 filed on September 28, 2005.

### Field of the Invention

The present invention relates to a disposable sanitary napkin that is intended to be worn in the undergarment of a user. The disposable sanitary napkin features a peelable protective layer which covers the adhesive fastener and which is of user-friendly design, thereby rendering it simpler to remove. The present invention also relates to a method for manufacturing a disposable sanitary napkin having such a protective layer.

### Background of the Invention

The term "sanitary napkin", as used herein, refers to an article that is worn by females in their undergarments, adjacent to the pudendal region, and which is intended to absorb and contain the various exudates that are discharged from the body (e.g., blood, menses, vaginal discharges, and urine). Hence, the term "sanitary napkin" encompasses pantiliners in addition to catamenial devices. The term "disposable" refers to articles that are intended to be discarded after a single use and preferably recycled, composted, or otherwise disposed of in an environmentally friendly manner. (That is, they are not intended to be laundered or otherwise restored or reused as an absorbent article.)

Disposable sanitary napkins are usually provided with an adhesive fastener intended to retain the sanitary napkin in place against the pudendal region of the wearer. To prevent soiling of the adhesive fastener and entanglement of the sanitary napkin prior to its use, the latter is manufactured with a peelable protective layer which covers the adhesive fastener. It is common to manufacture the protective layer from silicone-coated paper.

One drawback resulting from the above form of construction is the difficulty that users occasionally experience in removing the protective layer. A common approach is to insert a fingernail at the edge of the sanitary napkin between the protective layer and the surface of the napkin on which the adhesive fastener is applied. After the protective layer has been lifted off, the user can grasp it between her fingers and completely remove it from the adhesive fastener. The difficulty primarily resides in the insertion of the nail under the protective layer and the edge of the sanitary napkin. Sometimes this operation is difficult to accomplish and is therefore of some annoyance to the user.

Accordingly, there is a need in the industry to develop a novel sanitary napkin with a peelable protective layer that is easier to remove by comparison to prior art devices.

### Summary of the Invention

Under a first broad aspect, the invention provides a disposable sanitary napkin intended to be worn against a pudendal region of a wearer. The disposable sanitary napkin has a main body for absorbing bodily exudates, an adhesive fastener and at least one peelable protective layer. The main body includes an upper side facing toward the wearer when the sanitary napkin is in use, a lower side facing away from the wearer when the sanitary napkin is in use. An imaginary longitudinal centerline and a peripheral edge. The adhesive fastener is positioned on the main body and the peelable protective layer covers at least a portion of the adhesive fastener. At least one pull-tab projects beyond the peripheral edge of the main body and is offset from the imaginary longitudinal centerline. The pull tab contacts the peelable protective layer to promote separation of the peelable protective layer from the adhesive fastener when the pull tab is being pulled.

Under a second broad aspect, the invention provides a disposable sanitary napkin intended to be worn against a pudendal region of a wearer. The disposable sanitary napkin has a main body for absorbing bodily exudates, the main body including an upper side facing toward the wearer when the sanitary napkin is in use, a lower side facing away from the wearer when the sanitary napkin is in use and a a peripheral edge. An adhesive fastener is positioned on the main body. At least one peelable protective layer covers at least a portion of the adhesive fastener. The peelable protective layer includes at least one side edge having a portion which is co-terminus with a portion of the peripheral edge. At least one pull-tab projects beyond the peripheral edge of the main body. The pull tab contacts the peelable protective layer to promote separation of the peelable protective layer from the adhesive fastener when the pull tab is being pulled.

Under a third broad aspect, the invention provides a disposable sanitary napkin intended to be worn against a pudendal region of a wearer. The disposable sanitary napkin has a main body for absorbing bodily exudates. The main body includes an upper side facing toward the wearer when the sanitary napkin is in use, a lower side facing away from the wearer when the sanitary napkin is in use and an adhesive fastener on the upper side promoting adhesion of the main body to the user. The sanitary napkin also has at least one peelable protective layer covering at least a portion of the adhesive fastener. At least one pull-tab projects beyond the peripheral edge of the main body. The pull tab contacts the peelable protective layer to promote separation of the peelable protective layer from the adhesive fastener when the pull tab is being pulled.

Under a fourth broad aspect, the invention provides a method for manufacturing disposable sanitary napkins intended to be worn against a pudendal region of a user. The method includes providing a first continuous web, a second continuous web and a succession of absorbent systems at spaced-apart intervals between the first and second continuous webs. The first and second continuous webs are bonded around each absorbent system of the succession to form bonded regions associated with respective absorbent systems. The method includes effecting a first cut in each of the bonded regions and applying adhesive onto one of the first and second continuous webs such as to form adhesive-coated regions associated with respective absorbent systems. The method includes providing an expanse of peelable protective material over each adhesive-coated region and effecting a second cut around each absorbent system simultaneously through each of the bonded regions and the expanse of peelable protective material, the second cut intersecting the first cut. Such a method produces discrete sanitary napkins with peelable protective layers covering the respective adhesive-coated regions, where the second cut intersects the first cut.

### Brief Description of the Drawings

A detailed description of preferred embodiments of the present invention is provided herein below with reference to the following drawings, in which:
Figure 1 is a bottom plan view of a disposable sanitary napkin in accordance with a non-limiting example of implementation of the present invention;
Figure 2 is a bottom plan view of the disposable sanitary napkin shown in Figure 1, the figure showing the peelable protective layer in a partially removed state;
Figure 3 is a top plan view of the disposable sanitary napkin shown in Figure 1;
Figure 4 is a side view taken along line IV-IV of Figure 3;
Figure 5 schematically depicts the process used for manufacturing disposable sanitary napkins such as that shown in Figure 1;
Figure 5a is a magnified view of region A as shown in Figure 5;
Figure 5b is a magnified view of region B as shown in Figure 5;
Figure 5c is a magnified view of region C as shown in Figure 5;
Figure 5d is a magnified view of region D as shown in Figure 5;
Figure 5e is a magnified view of region E as shown in Figure 5; and
Figure 6 is a bottom plan view of a disposable sanitary napkin in accordance with a second non-limiting example of implementation of the present invention;
Figure 7 is a bottom plan view of the disposable sanitary napkin shown in Figure 6, the figure showing the peelable protective layer in a partially removed state;
Figure 8 is a top plan view of the disposable sanitary napkin shown in Figure 6;
Figure 9 schematically depicts the process used for manufacturing disposable sanitary napkins such as that shown in Figure 6;
Figure 9a is a magnified view of region A as shown in Figure 9;
Figure 9b is a magnified view of region B as shown in Figure 9;
Figure 9c is a magnified view of region C as shown in Figure 9; and
Figure 9d is a magnified view of region D as shown in Figure 9.
Figure 10 is is a bottom plan view of a disposable sanitary napkin similar to that of Figure 1 illustrating a variant of the peelable protective layer: and
Figure 11 is perspective view of a sanitary napkin according to a variant.

In the drawings, embodiments of the invention are illustrated by way of example. It is to be expressly understood that the description and the drawings are only for the purpose of illustration and as an aid to understanding. They are not intended to be a definition of the limits of the invention.

### Detailed Description of the Drawings

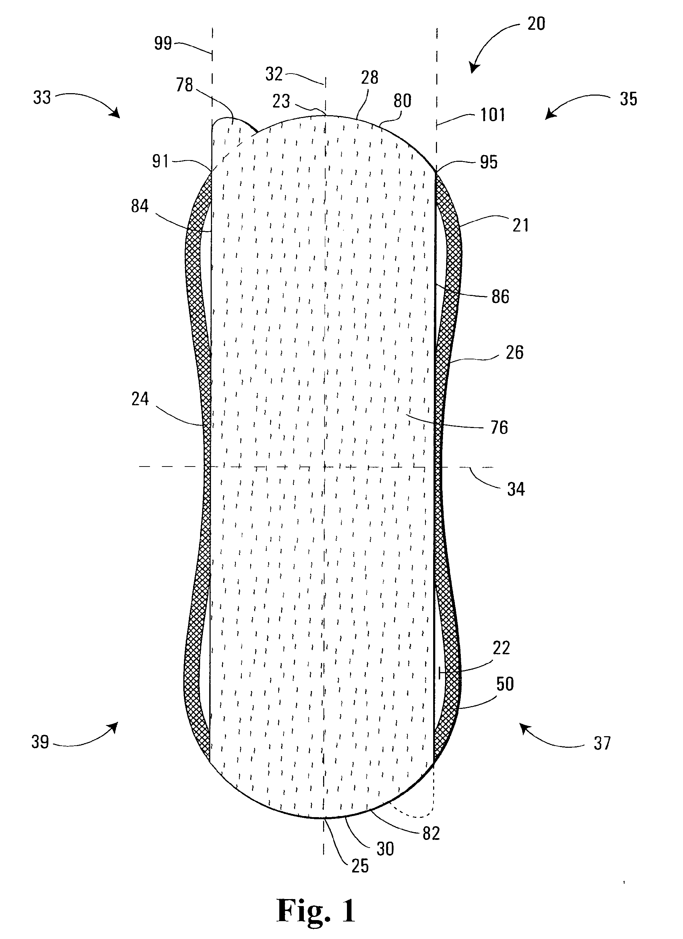

Shown in Figures 1 to 4 is a disposable sanitary napkin 20, in accordance with a specific non-limiting example of implementation of the present invention. Sanitary napkin 20 comprises a main body 22 featuring a peripheral edge 21 which is delimited by two generally opposing longitudinal side edges 24, 26 and two generally opposing transverse side edges 28, 30. As shown, an imaginary longitudinal centerline 32, which is generally equidistant from longitudinal side edges 24 and 26, runs down the center of the napkin 20 while an imaginary transverse centerline 34, which is perpendicular to the imaginary longitudinal centerline 32, runs across the napkin 20.

Note that the term "longitudinal"; as used herein, refers to a line, axis, or direction, in the plane of the sanitary napkin 20 that is generally aligned with (i.e.. approximately parallel to) a vertical plane which bisects a standing wearer into left and right body halves when the napkin is worn.

Together, the imaginary longitudinal and transverse centerlines 32 and 34 divide the napkin 20 into four quadrants 33, 35, 37, and 39. The figures also show that each transverse side edge 28 and 30 of napkin 20 respectively includes at least one apex area 23 and 25. The apex areas 23 and 25 are the areas of the transverse side edges which project the furthest from imaginary transverse centerline 34. It should be noted that the apex areas 23 and 25 can either be: a single point on the transverse side edges (as shown), a segment of the transverse side edges; or the entire transverse side edge, in the case where the side edge is generally rectilinear and parallel to the transverse centerline 34. Although the figures show that each transverse side edge 28, 30 respectively features a single apex area, it should also be understood that each transverse side edge 28, 30 may feature more than one apex area. This is the case, for example, when the transverse side edges 28, 30 have a wavy configuration. In the latter case, the apex areas are formed by the tips of the waves that are separated from one another by recessed portions.

It should also be noted that although the figures show the quadrants 33, 35, 37, and 39 as being of identical shape and size, quadrants of different sizes and shapes remain within the spirit of the present invention. Such would be the case, for example, if sanitary napkin 20 was an overnight napkin; the latter being asymmetrical with respect to the transverse centerline.

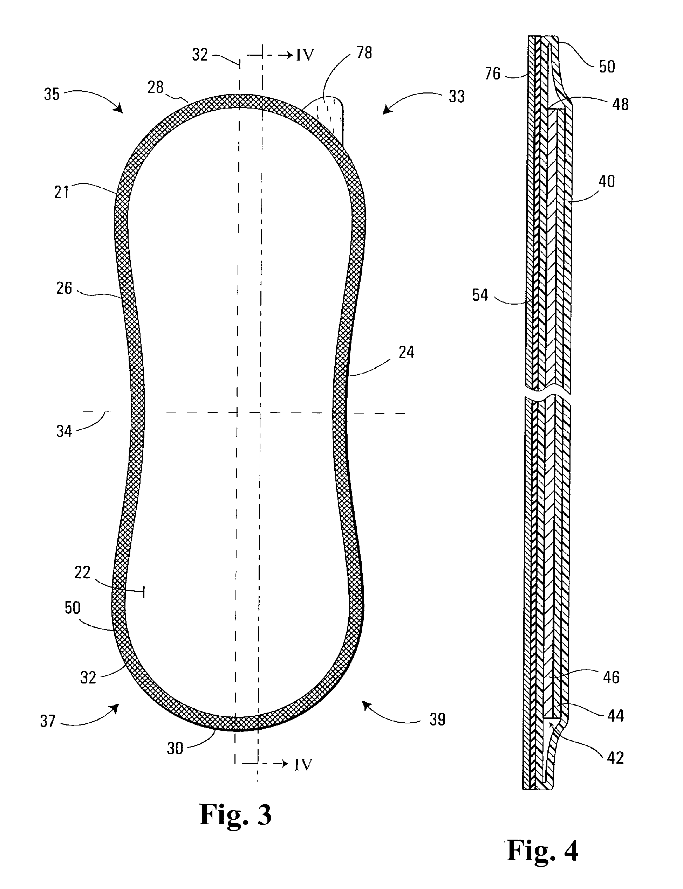

The main body 22 of sanitary napkin 20, as depicted in Figure 4, features a laminate structure including an upper side which will face the body of the wearer when the sanitary napkin is in use. The upper side includes a fluid-permeable cover layer 40. The main body 22 also includes a lower side that has a liquid-impervious barrier layer 48, which will face the environment (i.e., away from the body of the wearer, and in almost all cases, the wearer's undergarment) when the napkin is in use. The main body 22 further includes an absorbent system 42 between the fluid-permeable cover layer 40 and the liquid-impervious barrier layer 48. Absorbent system 42 may include a single layer or, alternatively, multiple layers. In the example of implementation depicted in Figure 4, absorbent system 42 has two components, namely a first absorbent layer 44 (commonly referred to as a "transfer layer") and a second absorbent layer 46 (commonly referred to as an "absorbent core''). Although not shown, a single layer, namely second absorbent layer 46, can form absorbent system 42.

The fluid-pervious cover layer 40 and the liquid-impervious barrier layer 48 are sealed together along their respective peripheral edges to form a peripheral flange seal 50. Preferably but not necessarily, the flange seal 50 extends continuously around the absorbent system 42 to completely enclose the latter. However, the present invention also contemplates embodiments where such is not the case.

Each of the above-mentioned layers will now be described in further detail.

### Fluid-Pervious Cover Layer

The fluid-pervious cover layer 40 is the top layer of the sanitary napkin 20. The purpose of the fluid-pervious cover layer 40 is to provide an interface that would normally contact the body of the wearer when the sanitary napkin 20 is in use. The cover layer 40 is porous to liquids since its main function is to capture a discharge of bodily exudate as quickly as possible and transfer it to the absorbent system 42 underneath.

Under one specific example of implementation, the fluid-pervious cover layer 40 is formed from an apertured thermoplastic film. Such films are common in the art. An example is the co-extruded film described in United States Patent 4,690,679, and marketed as RETICULON^{™} brand on sanitary napkins available from Johnson & Johnson Inc. of Montreal, Canada. Because of the high porosity of such films, they accomplish the function of quickly transferring body exudate to the inner layers (*i.e.,* the absorbent system 42) of the napkin 20.

The fluid-pervious cover layer 40 can also be made of fibrous materials, such as non-woven fibrous materials. The fluid-pervious cover layer 40 may be composed of only one type of fiber, such as polyester, or may be composed of bicomponent or conjugate fibers having a low melting point component and a high melting point component. Bicomponent fibers may be made up of a polyester core and a polyethylene sheath. The use of appropriate bicomponent materials results in a fusible non-woven fabric. Examples of such fusible fabrics are described in United States Patent 4,555,430. Using a fusible fabric increases the ease with which the fluid-pervious cover layer 40 may be mounted to the barrier layer 48 at the seal area 50.

The fibers may be selected from a variety of natural and synthetic materials such as nylon, polyester, rayon (in combination with other fibers), cotton acrylic fiber and the like and combinations thereof. An example is the multi-denier fluid-pervious cover layer described in the US patent application serial number 08/780,193 assigned to Johnson & Johnson. It will be evident to the person skilled in the art that a wide variety of other types of non-woven fabric materials can also be used.

### Absorbent System - First Absorbent Layer (Transfer Layer)

Adjacent to the fluid-pervious cover layer 40 on its inner side and bonded thereto is an optional fluid transfer layer 44 that may form part of the absorbent system 42. The transfer layer 44 provides the means of receiving body fluid from the fluid-pervious cover layer 40 and holding it until the highly-dense absorbent core 46 has an opportunity to absorb it.

The transfer layer 44 is preferably denser than than the fluid-pervious cover layer 40 and has a larger proportion of smaller pores than does the latter. These attributes allow the transfer layer 44 to contain body fluid and hold it away from the outer side of the fluid-pervious cover layer 40, thereby preventing the fluid from re-wetting the fluid-pervious cover layer 40 and its surface. However, the transfer layer 44 is preferably not so dense as to prevent the passage of the fluid through the transfer layer 44 and into the underlying absorbent core 46.

The transfer layer 44 may be composed of fibrous materials such as wood pulp, polyester, rayon, flexible foam, or the like, or combinations thereof. The transfer layer 44 may also comprise thermoplastic fibers for the purpose of stabilizing the layer and maintaining its structural integrity. The transfer layer 44 may be treated with surfactant on one or both of its sides in order to increase its wettability. However, the transfer layer 44 is generally relatively hydrophilic and may not require additional treatment. The transfer layer 44 is preferably bonded on both sides to the adjacent layers, i.e. the fluid-pervious cover layer 40 and the absorbent core 46. An example is the material sold by Merfin in the United-States under the commercial designation VICELL 6002.

### Absorbent System - Second Absorbent Layer (Absorbent Core)

Immediately adjacent to and bonded to the transfer layer 44 is the absorbent core 46; the latter also forming part of the absorbent system 42. The absorbent system 42 may comprise only the absorbent core 46 or it may comprise a plurality of layers, such as the absorbent core 46 in combination with the transfer layer 44 or any other additional layer. This is to say that the transfer layer 44 is not an essential component of the present invention.

The absorbent core 46 is a highly dense layer having a fine porosity. It has a large liquid-holding capacity and it is extremely retentive. Preferably, the absorbent core 46 comprises a pulp fluff material and may optionally include other absorbent materials or non-absorbent materials such as conjugate fibers, fusible fibers, binders, sphagnum moss, superabsorbents, and the like and combinations thereof. A suitable absorbent core 46 is described in the US patent 5,866,242 granted on February 2, 1999 to Tan et al. The contents of this document are hereby incorporated by reference.

### Liquid-Impervious Barrier Layer

Underlying the absorbent system 42 is a liquid-impervious barrier layer 48 comprising liquid-impervious film material to prevent liquid that is entrapped in the absorbent core 46 from egressing the sanitary napkin 20 and staining the wearer's undergarment. The liquid-impervious barrier layer 48 can be made of polymeric film, such as polyethylene or a polyethylene/ethylvinyl acetate (EVA), which is both inexpensive and readily available. The polymeric film is capable of fully blocking the passage of liquid or gas that may emanate from the absorbent system 42. In a variant, breathable films may be used that allow passage of gases while blocking liquid. A suitable example is a combination polyethylene/ethylvinyl acetate (EVA) film sold by the Edison Plastics Company in the United States under the commercial designation XP-1167B.

### Adhesives

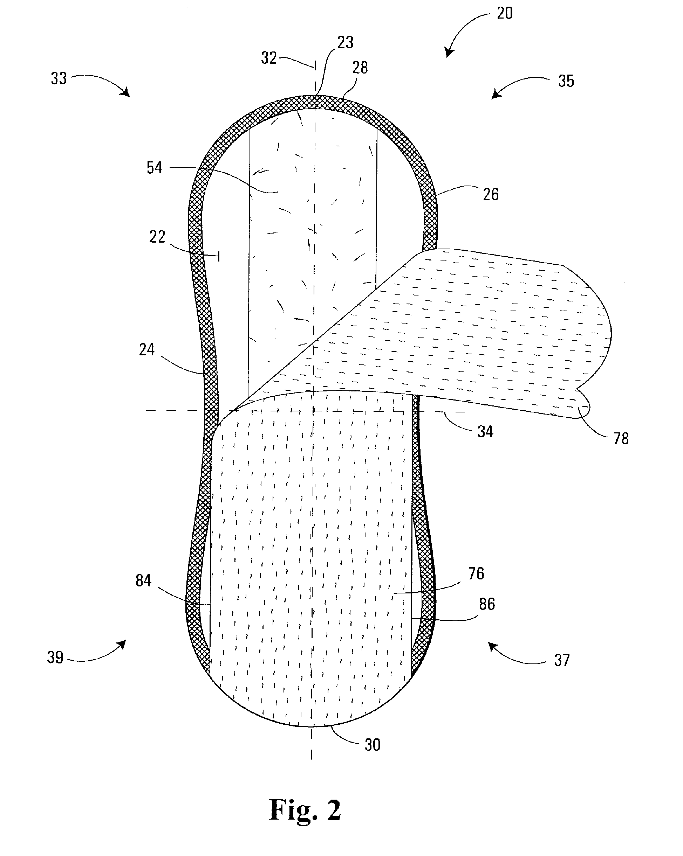

As shown in Figure 2, in order to secure the napkin 20 to the undergarment of a wearer, the liquid-impervious barrier layer 48 is provided with an adhesive fastener 54. Adhesive fastener 54 includes a layer of adhesive material on the environmental (i.e., undergarment) facing surface thereof. A suitable adhesive is the composition designated HL-1491 XZP commercially available from H.B. Fuller Canada, Toronto, Ontario, Canada. The adhesive fastener 54 may be applied to the liquid-impervious barrier layer in various patterns including: complete coverage in the form of a block; parallel longitudinal lines; parallel transverse lines; a line of adhesive following the perimeter of the structure; or the like. In the example shown in Figure 2, the adhesive fastener 54 is in the form of a continuous block.

### Protective Layer

A single protective layer 76, which is typically, but not necessarily, made of silicone-coated paper made from wet-laid Kraft wood pulp of a type well known in the art, covers the adhesive fastener 54 at least in part.

As shown in Figure 1, protective layer 76 includes a pair of generally opposite transverse side edges 80, 82, which are typically co-terminus with a portion of the corresponding transverse side edges 28, 30 of napkin 20, and a pair of generally opposite longitudinal side edges 84, 86; the latter being typically contained within the limits of the corresponding longitudinal side edges 24, 26 of napkin 20. Figure 1 further shows that each longitudinal side edge 84, 86 of protective layer 76 includes an apex 91 and 95. Apexes 91, 95 are the portions of the side edges 84, 86 that are the furthest from the imaginary longitudinal centerline 32. Apexes 91, 95 may be formed by a single point on the longitudinal side edges 84, 86, or the entire side edges 84, 86 when every point thereon is at the same distance from the longitudinal centerline. The latter embodiment is shown at Figure 1. Also, the longitudinal side edges 84, 86 may include several apexes which are at the same distance from the imaginary longitudinal centerline 32.

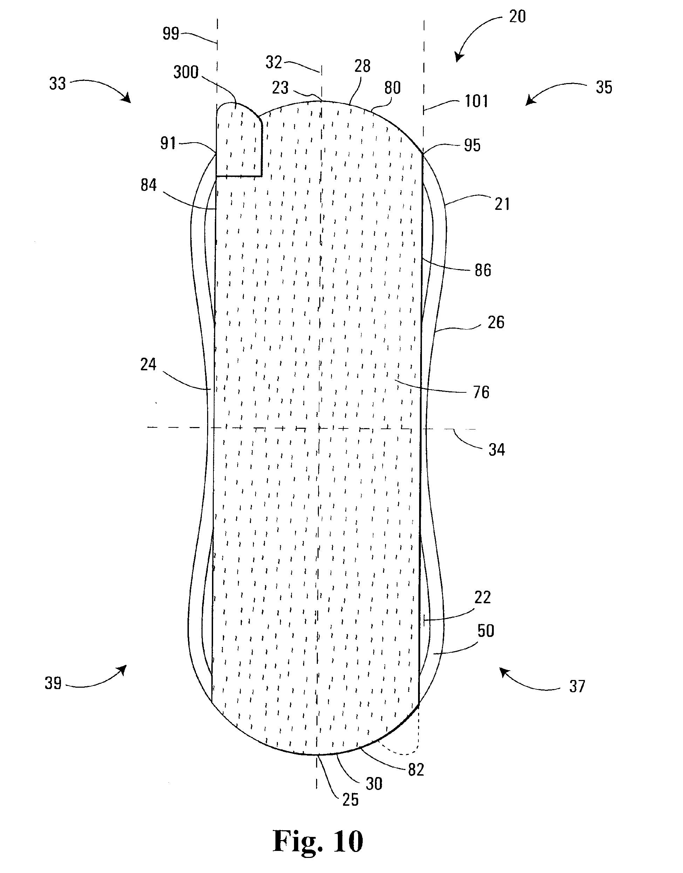

Protective layer 76, as shown in Figures 1 and 2, features a pull-tab 78, which in the specific embodiment shown, projects from transversely extending side edge 80 beyond the peripheral edge 21 of napkin 20. Consequently, a user may conveniently grasp pull-tab 78 between her fingers and subsequently peel back the protective layer 76, thereby exposing adhesive fastener 54. In the example of implementation shown in Figures 1 to 4, pull-tab 78 is formed integrally with the remainder of protective layer 76 and is adjacent to the portion of the transverse side edge 80 that is co-terminus with a portion of the corresponding transverse side edge 28 of the napkin 20. Alternatively, the pull-tab 78 can be formed independently and subsequently attached to the remainder of the protective layer 76 by any suitable means (e.g., adhesives and the like). This embodiment is shown at Figure 10. The pull tab 300 is in the form of a strip of material that is adhered to the protective layer 76.

The position of the pull-tab 78 in relation with the sanitary napkin's main body 22 can vary without departing from the spirit of the invention.

Under a first possibility, pull-tab 78 is offset with respect to the imaginary longitudinal centerline 32. This means that the imaginary longitudinal centerline 32 does not intersect pull-tab 78. The pull-tab 78 is also considered offset with respect to the imaginary longitudinal centerline 32 in the case when the imaginary longitudinal centerline 32 is coincident with an edge portion of pull-tab 78.

In a second possibility, pull-tab 78 is entirely contained within quadrant 33. This means that the pull-tab is not only offset with respect to the imaginary longitudinal centerline 32 but also with respect to the imaginary transverse centerline 34, thereby implying that the imaginary longitudinal and transverse centerlines 32 and 34 do not intersect pull-tab 78. The pull-tab 78 is also considered offset with respect to the imaginary longitudinal and transverse centerlines 32, 34 in the case when the imaginary longitudinal centerline 32 or the imaginary transverse centerline 34 is coincident with an edge portion of pull-tab 78.

In a third possibility, pull-tab 78 is located between an imaginary reference line 99 (shown in Figure 1) and the apex area 23. The imaginary reference line 99 is an imaginary line parallel to the imaginary longitudinal centerline 32 that intersects (or extends along) an apex 91 or 95. The pull-tab 78 may have an edge portion which is coincident with either or both of imaginary reference line 99 or apex area 23.

The embodiment of Figure 1 illustrates all three possibilities where pull-tab 78 is simultaneously: offset with respect to the imaginary longitudinal centerline 32; entirely contained within quadrant 33; and located between imaginary reference line 99 and apex area 23. However, it should be expressly noted that an embodiment that satisfies only one of the above possibilities falls within the scope of the present invention.

Although the figures show that pull-tab 78 is entirely contained within quadrant 33, it should be understood that a pull-tab 78 located in any other quadrant remains within the scope of the present invention.

In Figures 1-3, pull-tab 78 is shown as being shaped as a projection having a generally rounded end portion. It should be expressly understood, however, that pull-tabs of alternative shapes do not detract from the spirit of the present invention.

Figures 1 to 3 depict a sanitary napkin 20 featuring a single pull-tab 78. Alternatively, sanitary napkin 20 may also feature more than one pull-tab. Such a variant is shown, for example, in dotted lines in Figure 1.

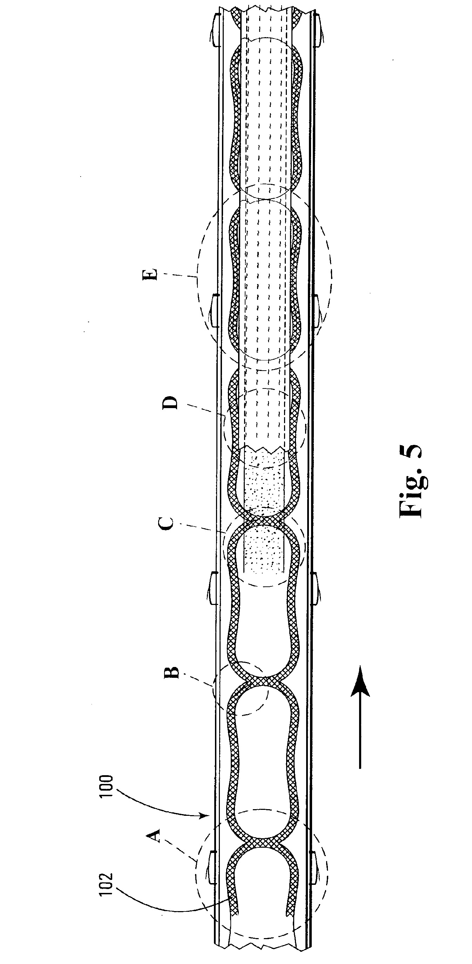

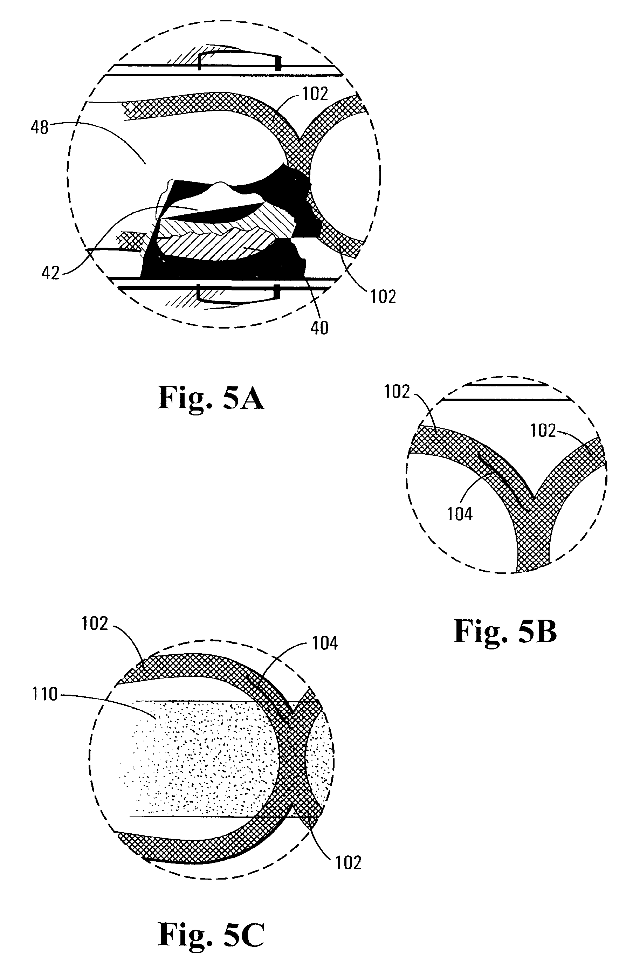

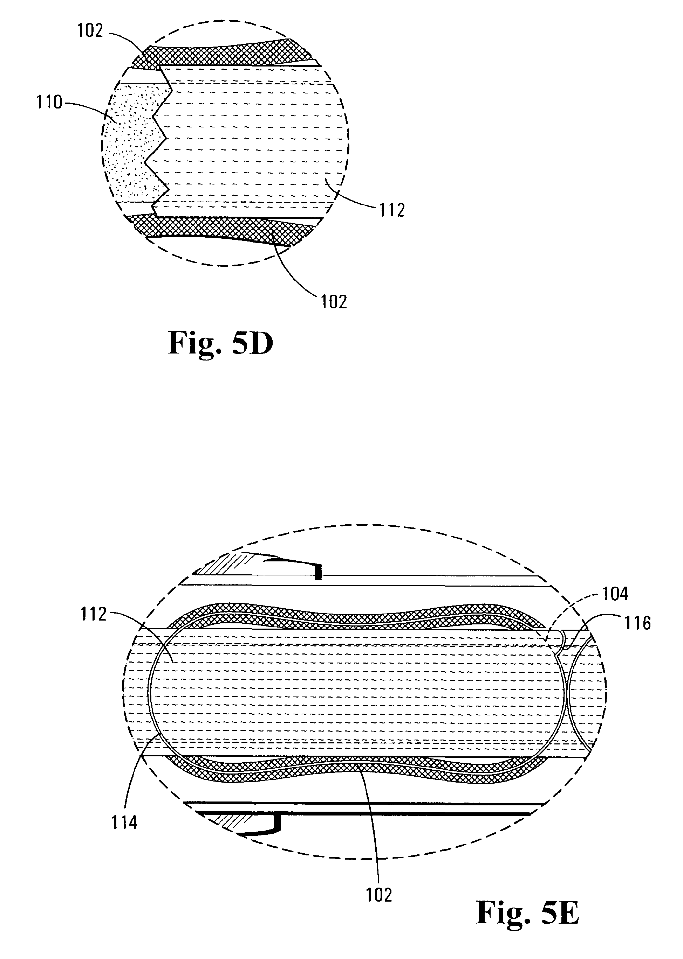

Figure 5 schematically depicts a method for fabricating the sanitary napkin 20 of Figures 1 to 4.

As shown at a first station denoted A, the method initially includes providing a bottom continuous web of the material for the fluid-permeable cover layer 40 and placing on top of this a succession of absorbent systems 42. A top continuous web of the material for the liquid-impervious barrier layer 48 is then placed over the succession of absorbent systems 42. The top and bottom continuous webs are bonded to one another around each absorbent system 42 to form bonded regions 102, as shown in cross-hatching in Figures 5 and 5A. Note that each bonded region 102 encloses an individual absorbent system 42 and generally resembles the final shape of a sanitary napkin. In order to effect the bonding, an apparatus that includes an anvil roll and a pattern roll, which define a nip therebetween, is generally used. Along its periphery, the pattern roll features one or more projections having an outline similar to the bonded regions 102 of Figure 5A.

At a second station denoted B, shown in more detail in Figure 5B, a small localized cut 104 is effected in a specific area of each bonded region 102. Cut 104 is generally effected in the middle of each bonded region 102 and is located in the vicinity of where pull-tab 78 will eventually be situated. In order to effect the cut 104, an apparatus having an anvil roll and a cutting roll, through which the top and bottom continuous webs pass, is typically used. The cutting roll has on its periphery cutting blades that perform the cutting of the bonded region 102 as the cutting roll turns. The shape of the cutting blades is such that as the rolls turn, they sever the top and bottom continuous webs only at the areas 104.

At a subsequent station denoted C, an adhesive-coated region 110, which will eventually form the adhesive fastener 54, is applied on the top continuous web. Preferably, a controlled coat applicator including a spray slot nozzle is used. Although Figure 5C shows that the adhesive-coated region 110 is continuous, it could also be intermittent. As shown in Figure 5D, an expanse of peelable protective material 112, which will eventually form the peelable protective layer 76, is subsequently applied onto the adhesive-coated regions 110. In the example shown, the expanse of peelable protective material 112 is in the form of a continuous web covering the adhesive-coated regions of a plurality of absorbent systems. Alternatively, a discrete expanse of peelable protective material is placed individually on each adhesive-coated region.

Although Figures 5C and 5D show that the adhesive-coated region 110 is directly applied onto the top of the continuous web prior to the application of peelable protective material 112, it should be understood that adhesive material may first be applied onto the expanse of peelable protective material 112 and then transferred onto the top continuous web as the expanse of peelable protective material 112 is applied to the top continuous web.

At a final processing station denoted E, the top and bottom continuous webs are cut at the areas 114, as shown in Figure 5E, to provide discrete sanitary napkins 20. The cutting can be done with any suitable means. A possible example is a cutting station including a cutting roll and an anvil roll through which the top continuous web, the bottom continuous web, the absorbent systems 202 and the expanse of peelable protective material pass. The cutting roll has on its periphery cutting blades that perform the cutting as the cutting roll turns. The shape of the cutting blades is such that as the rolls turn, they cut only at the areas 114.

As shown in Figure 5E, each cutting area 114 is generally continuous around the periphery defined by bonded region 102and includes a projecting area 116, which will eventually form pull-tab 78 and which generally registers with localized cut 104.

The cutting operation forming the cutting area 114 is such that the cutting area 114 will intersect the first cut 104. This is done such that all excess material that is outside the peripheral edge 21 will be allowed to fully separate from the final sanitary napkin. This excess material forms production waste and can be discarded or recycled. In the example shown in the drawings, the cutting area 114 intersects the cut 104 at two points that are spaced apart from one another.

Note that the small piece of excess material which registers with the tab 78, that is defined between the cut 104 and the cutting area 114 may remain attached to the tab 78 after the cut, due to the fact that the cut completely separates it from the remainder of the excess material and also because some adhesive is present between that small piece of excess material and the tab 78. However, this does not present a problem since the small piece of excess material, which follows the contour of the pull-tab 78 would then be functionally part of the pull tab 78. The user is then able to pull on the two-layer tab to peel away the peelable layer 112.

Although the above-described method yields a sanitary napkin 20 as shown in Figures 1-3, it should be apparent that slight modifications (i.e., having two localized cuts 104, having differently shaped cutting areas 114 including two projection areas 116, etc.) may be brought to the method in order to yield a napkin 20 having two or more pull-tabs 78, or other such variants.

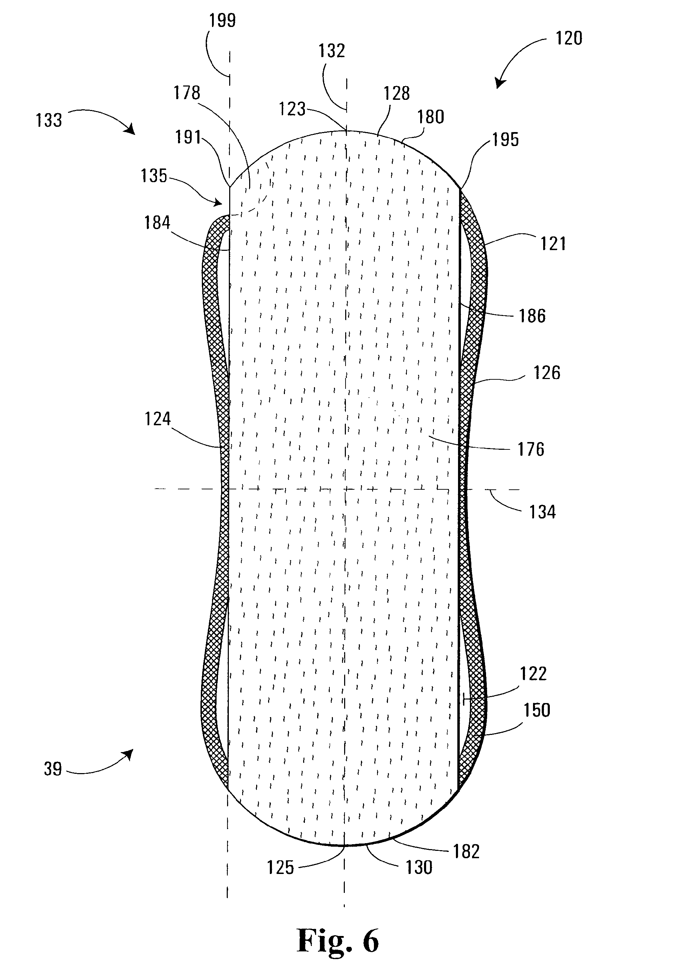

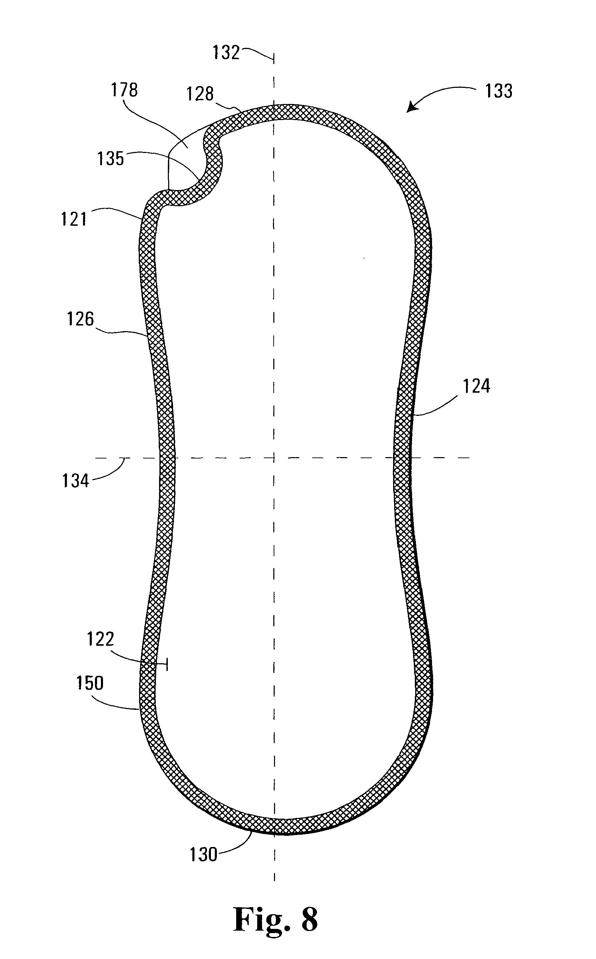

Shown in Figures 6 to 8 is a disposable sanitary napkin 120, in accordance with an alternative example of implementation of the present invention. As described above with respect to sanitary napkin 20, sanitary napkin 120 also comprises a main body 122 featuring a peripheral edge 121 which is delimited by two generally opposing longitudinal side edges 124, 126 and two generally opposing transverse side edges 128, 130. As will be described in more detail further on, the peripheral edge 121 of transverse edge 128 defines a notch 135 over which the protective layer 176 projects in order to form a pull-tab 178.

Sanitary napkin 120 further includes an imaginary longitudinal centerline 132, which is generally equidistant from longitudinal side edges 124 and 126, runs down the center of the napkin 120 while an imaginary transverse centerline 134, which is perpendicular to the imaginary longitudinal centerline 132, runs across the napkin 120.

Each transverse side edge 128 and 130 of napkin 120 respectively includes at least one apex area 123 and 125. The apex areas 123 and 125 are the areas of the transverse side edges, which project the furthest from imaginary transverse centerline 134.

The main body 122 of sanitary napkin 120 features a laminate structure that is the same as the laminate structure described above with respect to Figure 4. As such, sanitary napkin 20 includes a fluid-permeable cover layer 40, a liquid-impervious barrier layer 48 and an absorbent system 42 therebetween.

The fluid-pervious cover layer 40 and the liquid-impervious barrier layer 48 are sealed together along their respective peripheral edges to form a peripheral flange seal 150, as shown in Figure 6. Preferably but not necessarily, the flange seal 150 extends continuously around the absorbent system 42 to completely enclose the latter. In addition, the flange seal 150 follows the contour of the peripheral edge 121 at the area where it defines the notch 135. As such, the width of the flange seal 150 is substantially the same.

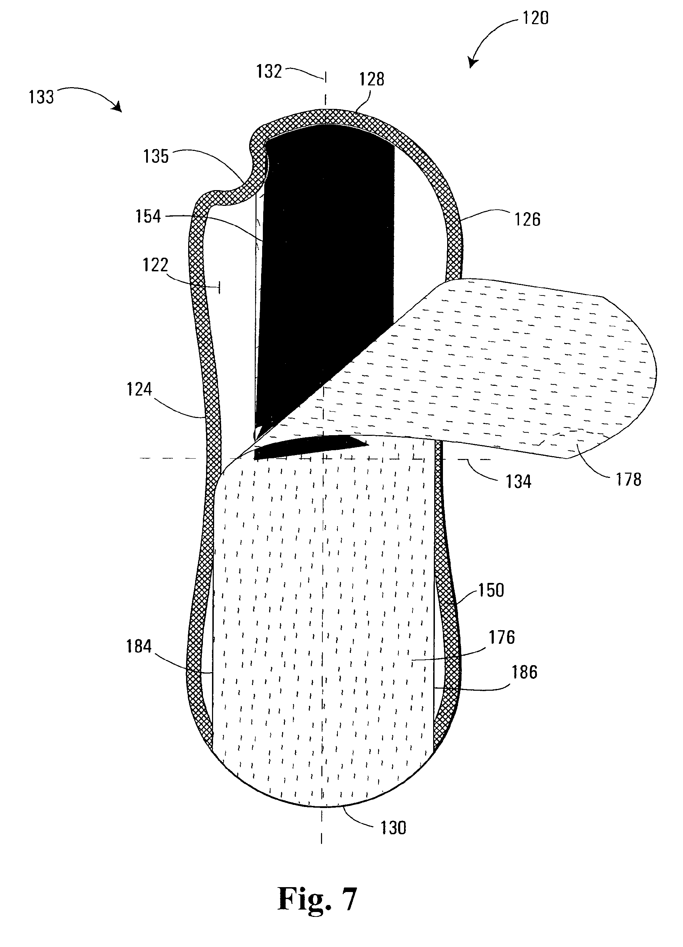

As shown in Figure 7, in order to secure the napkin 120 to the undergarment of a wearer, the napkin 120 includes an adhesive fastener 154 and a single protective layer 176 of material well known in the art for covering the adhesive fastener 154. As shown in Figure 6, the protective layer 176 includes a pair of generally opposite transverse side edges 180, 182 that each have a portion that is co-terminus with a portion of the corresponding transverse side edges 128, 130 of napkin 120. The protective layer also includes a pair of generally opposite longitudinal side edges 184, 186; the latter being typically contained within the limits of the corresponding longitudinal side edges 124, 126 of napkin 120.

As shown in Figures 6 and 7, protective layer 176 features a pull-tab 178, which in the specific embodiment shown, projects beyond the portion of the peripheral edge 21 of napkin 20 that defines notch 135. Consequently, the portion of the protective layer 176 that projects beyond the peripheral edge 121 forms a pull-tab 178 that a user may conveniently grasp between her fingers at the area where the notch 135 is located in order to peel back the protective layer 176 and expose adhesive fastener 154. Pull-tab 178 can be formed integrally with the remainder of protective layer 176 or can be formed independently and subsequently attached to the remainder of the protective layer 176 by any suitable means (e.g., adhesives and the like). Typically, the pull-tab 178 is adjacent to the portion of the transverse side edge 180 that is co-terminus with a portion of the corresponding transverse side edge 128 of the napkin 120.

As described above with respect to pull-tab 78, the position of the pull-tab 178 in relation with the sanitary napkin's main body 122 can be defined according to a number of different aspects. In a first aspect, pull-tab 178 is offset with respect to the imaginary longitudinal centerline 132. In a second aspect, pull-tab 178 is entirely contained within the quadrant 133, which means that the pull-tab is not only offset with respect to the imaginary longitudinal centerline 132, but also with respect to the imaginary transverse centerline 134. In a third aspect, pull-tab 178 is located between an imaginary reference line 199 (shown in Figure 6) and the apex area 123. The imaginary reference line 199 is an imaginary line parallel to the imaginary longitudinal centerline 132 that intersects an apex 191 or 195. Apexes 191, 195 are the portions of the side edges 184, 186 that are the furthest from the imaginary longitudinal centerline 132. Apexes 191, 195 may be formed by a single point on the longitudinal side edges 184, 186 or the entire side edges 184, 186 when every point thereon is at the same distance from the longitudinal centerline, which is the case shown in Figure 6.

The embodiment of Figure 6 illustrates all three aspects where pull-tab 178 is simultaneously: offset with respect to the imaginary longitudinal centerline 132; entirely contained within quadrant 133; and located between imaginary reference line 191 and apex area 132. However, it should be expressly noted that an embodiment that satisfies only one of the above aspects falls within the scope of the present invention.

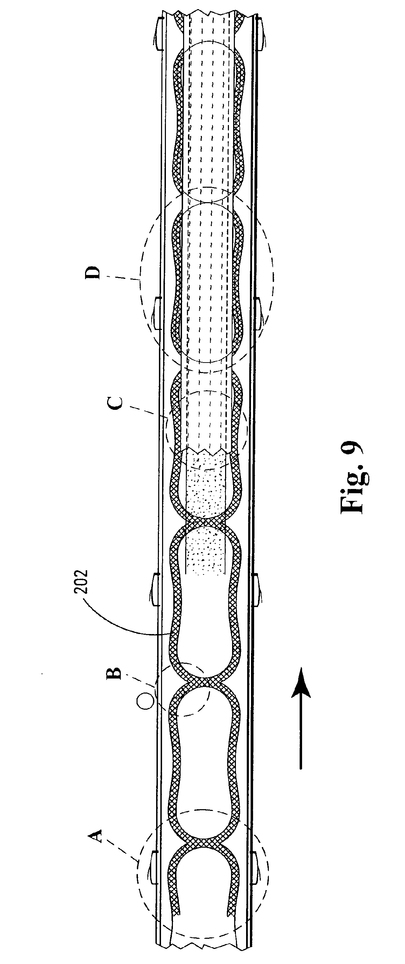

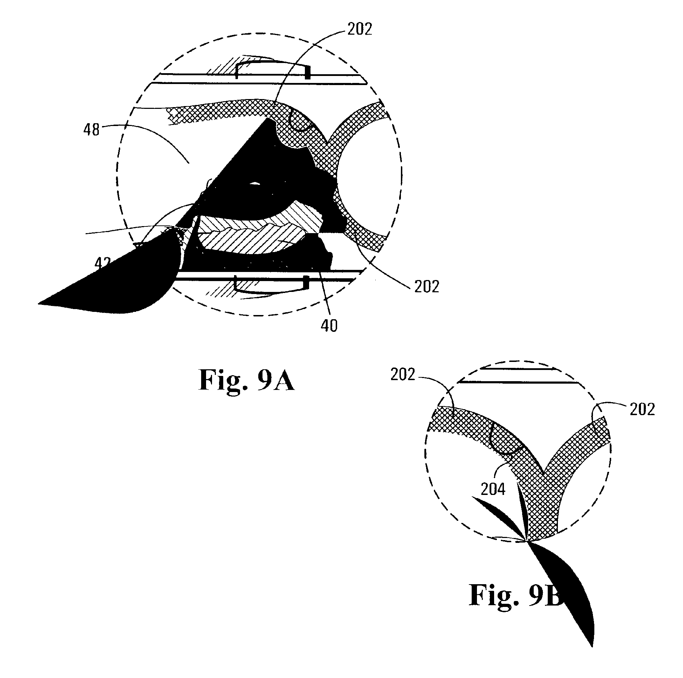

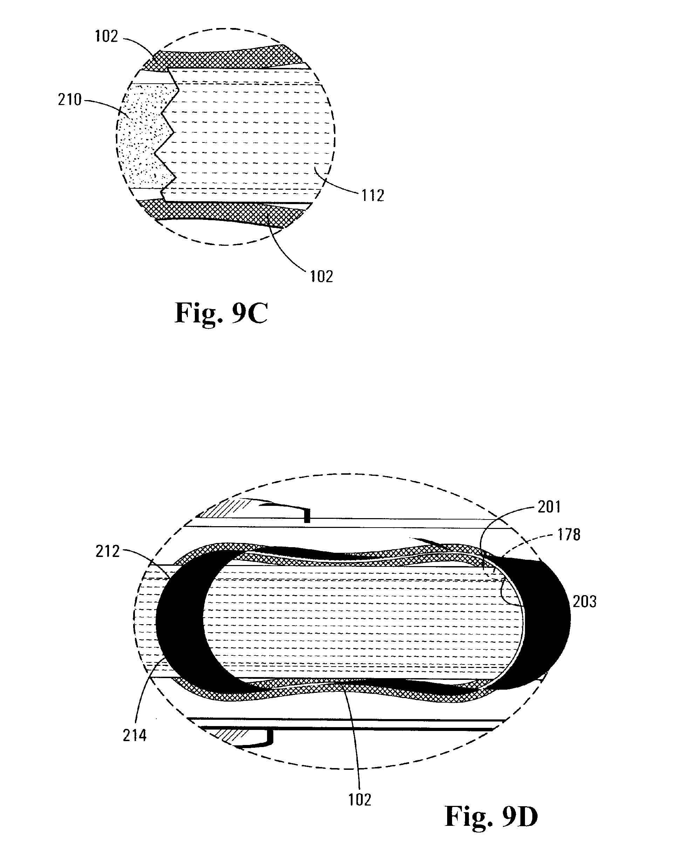

Figure 9 schematically depicts a method for fabricating the sanitary napkin 120 of Figures 6 to 8. The method is similar to the method described above with respect to figures 5 and 5A-5C, however, the shape of the bonded regions 202 and the first and second cuts are different. As shown in the Figures 9 and 9A each bonded region 202 shown in cross-hatching encloses an individual absorbent system 42 and generally resembles the final shape of a sanitary napkin, including the portion of the peripheral edge 121 that defines the notch 135.

At a second station denoted B, a small localized cut 204 is effected in a specific area of each bonded region 202 at the location where the notch 135 will be defined. As shown in Figure 9B, cut 204 is generally a U-shaped cut that commences at the edges of the bonded region 202 and dips down such that the base of the U-shaped cut is effected in the area of the bonded region 202 that will eventually form the contour of the notch 135. Cut 204 is located in the vicinity of where pull-tab 178 will eventually be situated. In order to effect the cut 204, an apparatus having an anvil roll and a cutting roll, through which the top and bottom continuous webs pass, is typically used. The cutting roll has on its periphery cutting blades that perform the cutting of the bonded region 202 as the cutting roll turns. The shape of the cutting blades is such that as the rolls turn, they sever the top and bottom continuous webs only at the areas 204.

At a subsequent station denoted C, an adhesive-coated region 210, which will eventually form the adhesive fastener 154, is applied on the top continuous web. In addition, an expanse of peelable protective material 212, which will eventually form the peelable protective layer 176, is subsequently applied onto the adhesive-coated regions 210. In the example shown, the expanse of peelable protective material 212 is in the form of a continuous web covering the adhesive-coated regions 210.

At a final processing station denoted D, the top and bottom continuous webs are cut at the areas 214, as shown in Figure 9D, to provide discrete sanitary napkins 120. The cutting can be done with any suitable means. A possible example is a cutting station including a cutting roll and an anvil roll wherein the cutting roll has on its periphery cutting blades that perform the cutting as the cutting roll turns. The shape of the cutting blades is such that as the rolls turn, they cut only at the areas 214.

Note that the small piece of excess material that registers with the tab 178, that is defined between the cut 204 and the cutting area 214 may remain attached to the tab 178 after the cut, due to the fact that the cut completely separates it from the remainder of the excess material and also because some adhesive is present between that small piece of excess material and the tab 178. However, this does not present a problem since the small piece of excess material, which follows the contour of the pull-tab 178 would then be functionally part of the pull tab 178. The user is then able to pull on the two-layer tab to peel away the peelable layer 212.

As shown in Figure 9D, each cutting area 214 is generally continuous around the periphery defined by bonded region 202. In addition, the cutting area 214 intersects with the U-shaped localized cut 204 at two points 201 and 203 that are spaced apart from each other such that the bonded area 202 within the boundaries of the U-shaped cut drops away and leaves the section of the protective layer 176 that projects over portion of the peripheral edge 121 that defines the notch 135 with no bonded region underneath it. It is this section of the protective layer 176 that forms pull-tab 178.

Although the above-described method yields a sanitary napkin 20 as shown in Figures 6-8, it should be apparent that slight modifications (i.e., having two localized cuts 204, having differently shaped cutting areas 214 including two projection areas) may be brought to the method in order to yield a napkin 120 having two or more pull-tabs 178, or other such variants.

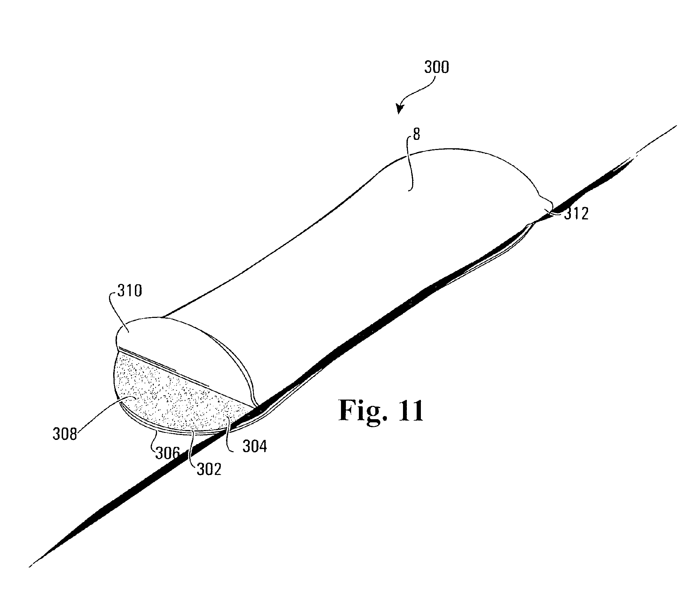

Figure I 1 illustrates a variant. The sanitary napkin 300 has a main body 302 including an upper side 304 that in use faces the wearer. The upper side 304 includes a fluid-permeable cover layer. In the example shown in Figure 11, the fluid permeable cover layer extends over the entirety of the upper side 304. It will be plain that this feature is not an absolute requirement. Embodiments where the permeable cover layer extends over a limited portion of the upper side 304 can be envisaged without departing from the spirit of the invention. The main body 302 also includes a lower side 306 that is capable of blocking the egress of fluid from the absorbent system of the sanitary napkin 300. As it is apparent from the drawings, the lower side 306 faces away from the wearer when the sanitary napkin 300 is in use. The absorbent system of the sanitary napkin 300 can be constructed in an identical or similar fashion to the absorbent systems described earlier.

A layer of adhesive 308 is is provided on the upper side of the main body 302 to promote adhesion of the sanitary napkin 300 to the skin of the wearer. An example of a suitable adhesive is described in the US patent application XXXXXX, filed on YYYYYY and owned by ZZZZZ, the contents of which are hereby incorporated by reference.

The adhesive 308 is covered by a peelable protective layer 310 that has a pull-tab 312 to assist the wearer during the removal of the peelable protective layer 310. The pull-tab contacts the peelable protective layer 310 such that when the pull-tab 312 is pulled by the wearer, the pulling action with cause the peelable protective layer to separate from the upper side 304. The protective layer 310 and the pull tab 312 can be made in the same way described in connection with the previous embodiments. Specifically, the pull tab 312 can be made integrally with the peelable protective layer 310, which is the case shown in Figure 11 or it may be made separately and then associated with the peelable protective layer 310.

Although various embodiments have been illustrated, this was for the purpose of describing, but not limiting, the invention. Various modifications will become apparent to those skilled in the art and are within the scope of this invention, which is defined more particularly by the attached claims.

## Claims

1. A disposable sanitary napkin intended to be worn against a pudendal region of a wearer, said disposable sanitary napkin comprising:
- a main body for absorbing bodily exudates, said main body including:
a) an upper side facing toward the wearer when the sanitary napkin is in use;
b) a lower side facing away from the wearer when the sanitary napkin is in use;
c) an imaginary longitudinal centerline; and
d) a peripheral edge;
- an adhesive fastener on said main body;
- at least one peelable protective layer covering at least a portion of said adhesive fastener;
- at least one pull-tab projecting beyond said peripheral edge and being offset with respect to said imaginary longitudinal centerline, said pull tab contacting said peelable protective layer to promote separation of said peelable protective layer from said adhesive fastener when said pull tab is being pulled.

2. A disposable sanitary napkin as defined in claim 1, wherein said peelable protective layer includes at least one side edge having a portion which is co-terminus with a portion of said peripheral edge.

3. A disposable sanitary napkin as defined in claim 2, wherein said pull-tab is adjacent to said portion of said peelable protective layer that is co-terminus with a portion of said peripheral edge.

4. A disposable sanitary napkin as defined in claim 3, wherein said pull-tab is integral with said peelable protective layer.

5. A disposable sanitary napkin as defined in claim 3, wherein said pull-tab and said peelable protective layer are distinct elements connected to one another.

6. A disposable sanitary napkin as defined in any of the preceding claims, wherein said adhesive fastener is applied on said lower side.

7. A disposable sanitary napkin as defined in any of claims 1 to 5, wherein said adhesive fastener is applied on said upper side.

8. A disposable sanitary napkin intended to be worn against a pudendal region of a wearer, said disposable sanitary napkin comprising:
- a main body for absorbing bodily exudates, said main body including:
a) an upper side facing toward the wearer when the sanitary napkin is in use;
b) a lower side facing away from the wearer when the sanitary napkin is in use;
c) a peripheral edge;
- an adhesive fastener on said main body;
- at least one peelable protective layer covering at least a portion of said adhesive fastener, said peelable protective layer including at least one side edge having a portion which is co-terminus with a portion of said peripheral edge;
- at least one pul1-tab projecting beyond said peripheral edge of said main body, said pull tab contacting said peelable protective layer to promote separation of said peelable protective layer from said adhesive fastener when said pull tab is being pulled.

9. A disposable sanitary napkin as defined in claim 8, wherein said pull-tab is adjacent the portion of the side edge which is co-terminus with a portion of said peripheral edge.

10. A disposable sanitary napkin as defined in claim 9, wherein said main body includes:
- a pair of generally opposite longitudinal side edges;
- a pair of generally opposite transverse side edges; and
said peelable protective layer further includes:
- a pair of generally opposite longitudinal side edges;
- a pair of generally opposite transverse side edges;
wherein at least a portion of a particular transverse side edge of said peelable protective layer is co-terminus with at least a portion of a corresponding transverse side edge of said main body.

11. A disposable sanitary napkin as defined in claim 10, wherein said at least one pull-tab projects from said particular transverse side edge of said peelable protective layer.

12. A disposable sanitary napkin as defined in claim 11, wherein at least a portion of a particular transverse side edge of said main body includes a notch.

13. A disposable sanitary napkin as defined in claim 12, wherein said pull-tab projects from said periphery in the region of said notch.

14. A disposable sanitary napkin as defined in any of claims 8 to 13, wherein said main body comprises a longitudinally extending centerline and a transversally extending centerline that divide said disposable sanitary napkin into four quadrants, said at least one pull-tab being contained entirely within one of said quadrants.

15. A disposable sanitary napkin as defined in any of claims 8 to 14, wherein said adhesive fastener is applied on said lower side.

16. A disposable sanitary napkin as defined in any of claims 8 to 14, wherein said adhesive fastener is applied on said upper side.

17. A method for manufacturing disposable sanitary napkins intended to be worn against a pudendal region of a user, said method comprising:
- providing a first continuous web, a second continuous web and a succession of absorbent systems at spaced-apart intervals between said first and second continuous webs, said first and second continuous webs being bonded around each absorbent system of said succession to form bonded regions associated with respective absorbent systems of said succession;
- effecting a first cut in each of said bonded regions;
- applying adhesive onto one of the first and second continuous webs such as to form adhesive-coated regions associated with respective absorbent systems of said succession;
- providing an expanse of peelable protective material over each adhesive-coated region;
- effecting a second cut around each absorbent system of said succession simultaneously through each of said bonded regions and said expanse of peelable protective material such as to produce discrete sanitary napkins with peelable protective layers covering the respective adhesive-coated regions at least in part, the second cut intersecting said first cut.

18. A method as defined in claim 17, wherein a discrete sanitary napkin produced by the second cut has a pull-tab.

19. A method as defined in claim 17 or claim 18, wherein said second cut intersects said first cut at two spaced-apart locations.

20. A method as defined in any of claims 17 to 19, wherein said expanse of peelable protective material is in the form of a continuous web covering the adhesive-coated regions of a plurality of absorbent systems.

21. A method as defined in any of claims 17 to 20, wherein a discrete expanse of peelable protective material is applied over each adhesive-coated region.

22. A method as defined in any of claims 17 to 21, wherein said first cut forms a notch in said bonded region.

23. A method as defined in any of claims 17 to 22, wherein said first cut forms a portion of a final peripheral edge of the disposable sanitary napkin.

24. A disposable sanitary napkin intended to be worn against a pudendal region of a wearer, said disposable sanitary napkin comprising:
- a main body for absorbing bodily exudates, said main body including:
a) an upper side facing toward the wearer when the sanitary napkin is in use;
b) a lower side facing away from the wearer when the sanitary napkin is in use;
- an adhesive fastener on said upper side promoting adhesion of said main body to the user ;
- at least one peelable protective layer covering at least a portion of said adhesive fastener;
- at least one pull-tab projecting beyond said peripheral edge of said main body, said pull tab contacting said peelable protective layer to promote separation of said peelable protective layer from said adhesive fastener when said pull tab is being pulled.

25. A disposable sanitary napkin as defined in claim 24, wherein said peelable protective layer includes at least one side edge having a portion which is co-terminus with a portion of said peripheral edge.

26. A disposable sanitary napkin as defined in claim 25, wherein said pull-tab is adjacent to said portion of said peelable protective layer that is co-terminus with a portion of said peripheral edge.

27. A disposable sanitary napkin as defined in claim 26, wherein said pull-tab is integral with said peelable protective layer.

28. A disposable sanitary napkin as defined in claim 26, wherein said pull-tab and said peelable protective layer are distinct elements connected to one another.

29. A disposable sanitary napkin as defined in any of claims 24 to 28, wherein said main body has an imaginary longitudinal centerline, said pull-tab being offset with respect to said imaginary centerline.
